(19) Europäisches Patentamt
European Patent Office
Office européen des brevets

(11) **EP 4 752 497 A1**

(12) **EUROPEAN PATENT APPLICATION**

(43) Date of publication:
**03.06.2026 Bulletin 2026/23**

(21) Application number: **24216386.3**

(22) Date of filing: **29.11.2024**

(51) International Patent Classification (IPC):
*G01B 9/02015* (2022.01)    *A61B 3/10* (2006.01)
*G01B 9/02* (2022.01)    *G01B 9/02091* (2022.01)

(52) Cooperative Patent Classification (CPC):
**G01B 9/02028; A61B 3/102; G01B 9/02027;
G01B 9/02051; G01B 9/02091;** G01B 9/0203

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Imec VZW
3001 Leuven (BE)**

(72) Inventors:
• **KIM, Joonyoung
  3000 Leuven (BE)**
• **JANSEN, Roelof
  3001 Heverlee (BE)**
• **ROTTENBERG, Xavier
  3010 Kessel-Lo (BE)**
• **VAN DORPE, Pol
  3510 Spalbeek (BE)**

(74) Representative: **AWA Sweden AB
Matrosgatan 1
Box 5117
200 71 Malmö (SE)**

(54) **A DEVICE, A SYSTEM, AND A METHOD FOR INTERFEROMETRY DETECTION**

(57)    A device (100; 200) for interferometry detection comprises: a receiving unit (114; 214) configured to receive a reference beam and a response beam, based on a sample interacting with the sample beam to form the response beam carrying information of the sample; at least a first waveguide system (120; 220) configured to receive the reference beam and configured to transport the reference beam along different path lengths to a plurality of first outputs (126; 226); a plurality of light sensors (130; 230) configured to receive a combination of the reference beam and the response beam forming an interference of the reference beam and the response beam, wherein the plurality of light sensors (130; 230) are configured to simultaneously detect interference of the reference beam and the response beam corresponding to different path length relations between the reference beam and the response beam.

Fig. 2

**(Cont. next page)**

*Fig. 3b*

**Description**

Technical field

The present description relates to interferometry detection. In particular, the present description relates to a device and a method for interferometry detection, and a system incorporating a plurality of interferometry detection units.

Background

Interferometry may be used for investigating an object, which may also be referred to herein as a sample.

For instance, a Michelson interferometer may be used, wherein a light signal is split into a reference beam and a sample beam traveling in two different paths, wherein the sample beam interacts with a sample. The reference beam and a response beam, formed by interaction of the sample beam with the sample, may thereafter be re-combined. An intensity of the interference between the reference beam and the response beam is dependent on a phase relation between reference beam and the response beam. The interference between the reference beam and the response beam may thus provide information of the sample.

In some applications, interferometry may be used for acquiring spatially resolved information from the sample. For instance, in optical coherence tomography (OCT), sample information may be acquired in three dimensions. OCT may be performed for analysis of biological tissue and is frequently used for analysis of a retina of a person.

In OCT, the sample beam may be focused onto a point of the retina. A path length traveled by the reference beam may be varied in time by scanning a position of a mirror used for reflecting the reference beam. This may allow determining depth information from the retina, as a different path length of the reference beam may be used for extracting information from different depths of the retina. The sample beam may further be scanned over different points of the retina in order to acquire information resolved over a surface of the retina. However, this implies that scanning in three dimensions (two-dimensional scanning of sample beam and one-dimensional scanning of reference beam) may be required for acquiring the three-dimensional information. Thus, the acquisition of three-dimensional information is time consuming as scanning in three dimensions is required. This may be particularly challenging for acquiring information of the retina, as the eye may need to be kept still during the OCT measurement.

In order to provide a faster OCT measurement, a full-field set-up may be used. A full-field OCT (FF-OCT) measurement may direct a sample beam onto the entire surface of the retina and a two-dimensional image of interference of the response beam and the reference beam may be acquired for representing the entire surface of the retina. Scanning in the path of the reference beam may still be needed for acquiring information of different depths of the retina. Thus, a speed of acquiring the three-dimensional information is still limited by the need for scanning. In addition, the need for a scanning mirror implies that a system for FF-OCT measurement may be bulky.

Further, the simultaneous acquisition of information from the entire surface of the retina implies that there is a risk of crosstalk between pixels. This implies that the FF-OCT measurement may provide lower image contrast and clarity compared to an OCT measurement using scanning of the sample beam.

Hence, there is a need for an improved speed in acquiring information of a sample based on interferometry. In addition, it would be advantageous to provide less bulky systems and to provide an improved contrast in acquisition of information.

Summary

An objective of the present description is to provide fast detection of information of a sample based on interferometry. Another objective of the present description is to provide interferometry detection using a compact system. Another objective of the present description is to provide interferometry detection with a high contrast.

These and other objectives are at least partly met by the invention as defined in the independent claims. Preferred embodiments are set out in the dependent claims.

According to a first aspect, there is provided a device for interferometry detection, said device comprising: a receiving unit configured to receive a reference beam and a response beam, wherein the response beam is a response to a sample beam formed by splitting a light signal into the reference beam and the sample beam, wherein the sample beam is directed towards a sample for interacting with the sample beam to form the response beam carrying information of the sample; at least a first waveguide system configured to receive a first optical signal, wherein the first optical signal is the reference beam or the response beam, wherein the first waveguide system is configured to transport the first optical signal along different path lengths to a plurality of first outputs; a plurality of light sensors, wherein each light sensor is configured to receive a combination of the reference beam and the response beam forming an interference of the reference beam and the response beam, wherein different light sensors of the plurality of light sensors are associated with different first outputs of the plurality of first outputs of the first waveguide system such that a path length of the reference beam in relation to a path length of the response beam is different for the different light sensors, wherein the plurality of light sensors are configured to simultaneously detect interference of the reference beam and the response beam corresponding to different path length relations.

The device is configured to transport the first optical signal along different path lengths to a plurality of first outputs. This implies that the device enables simultaneous detection of different path length relations between the reference beam and the response beam. Hence, instead of scanning a mirror in a path of the reference beam, the different path length relations may be provided by a physical arrangement of the at least first waveguide system.

The different light sensors are configured to receive signals in which different path length relations of the reference beam and the response beam are provided. This implies that the different light sensors are configured to determine information relating to different depths of the response beam in the sample.

The information relating to different depths may thus be simultaneously acquired. This implies that there is no need for a time resolved measurement for acquiring the information relating to different depths. Hence, the device allows very fast interferometry detection for acquiring depth information of a sample.

It is not necessary to use scanning of an optical component for acquiring information relating to different depths of the sample. This implies that a system for interferometry detection may be very compact.

It should be realized that the device for interferometry detection may relate to a single unit which may be configured to detect information from a single position of a surface of a sample. The device may be arranged in an array of devices, such that the array may be used for acquiring information from a plurality of positions of the surface. However, it should be realized that the device for interferometry detection need not necessarily be arranged in an array and may be used as a single measurement unit for acquiring information relating to different path length relations between the reference beam and the response beam for a single position of a sample.

The device is configured to perform interferometry detection by being configured such that the response beam and the reference beam interfere. The device may thus be configured to detect a light signal based on interference between the response beam and the reference beam. Each light sensor may for instance be configured to detect an intensity of light, which may be dependent on a phase relation (interference) between the response beam and the reference beam. Thus, the device is not configured to detect an interference pattern in space formed by the response beam and the reference beam but may rather be configured to detect an intensity value.

The reference beam and the sample beam may be formed by splitting of a light signal. The light signal may be any light signal, such as light from a broadband light source, which may have a short coherence length, such as a coherence length in micrometer scale.

The reference beam and the sample beam may be formed by a splitter, such as a beam splitter, which may be configured to split the light signal into a first fraction forming the reference beam and a second fraction forming the sample beam. The first and the second fraction may be equal and may be 50% of the light signal.

It should be realized that the splitter may form part of the device. However, the splitter may alternatively be external to the device. The sample beam may be output by the external splitter towards the sample but may preferably be output by the device towards the sample such that an accurate relation between the sample beam and the reference beam may be defined.

The receiving unit of the device may thus be defined by inputs in which the reference beam and the response beam, respectively, are received. The reference beam may be received in an optical waveguide forming the input for receiving the reference beam. This optical waveguide may be directly connected to the splitter for receiving the reference beam or may be otherwise connected for receiving the reference beam.

The response beam may be received at an input configured to couple in light from the sample.

According to an embodiment, the device comprises an output coupler for outputting the sample beam towards a spot on the sample and the device comprises an input coupler configured to couple in light from the spot on the sample. The output coupler and the input coupler may be formed, respectively, by grating couplers.

This implies that the output coupler and the input coupler may together function as a detector pinhole. Thus, the device may be configured to provide a confocal property for interferometry detection. This implies that crosstalk from adjacent portions on the sample may be reduced and that information from the spot on the sample towards which the sample beam is directed may be accurately detected by the device. In particular, the confocal property for interferometry detection may allow out-of-focus light to be rejected.

The device may be formed in a compact arrangement. For instance, the device may be formed in a photonic integrated circuit. The device may be arranged in a system. The system may include a light source for generating a light signal, and a splitter for splitting the light signal into the reference beam and the sample beam. The light source and/or the splitter could be external to a photonic integrated circuit but may alternatively be integrated with the receiving unit, the first waveguide system and the plurality of light sensors. In addition, it should be realized that a light source and/or a splitter may be shared by a plurality of parallel devices for interferometry detection.

According to yet another alternative, components of the device, including the receiving unit, the first waveguide system and the plurality of light sensors, may be formed as separate physical units with a well-defined interrelation between the components for ensuring an appropriate function of the device.

The sample beam may be provided for causing an interaction between the sample beam and the sample.

The sample may for instance be configured to back-scatter the sample beam to form the response beam. However, it should be realized that the sample may alternatively interact with the sample beam in a different manner to form the response beam. For instance, the sample beam may be reflected by the sample to form the response beam. Alternatively, the sample beam may be absorbed by the sample, wherein the response beam may correspond to transmission of the sample beam through the sample.

The first waveguide system may be formed by one or more waveguides configured to transport the first optical signal in the one or more waveguides. The first optical signal may follow different paths in different waveguides for being provided to the plurality of first outputs. Alternatively, or additionally, the first optical signal may be propagated to first outputs in a sequence, such that the first optical signal passes one first output in the sequence before reaching another first output in the sequence.

The first waveguide system may be configured such that portions of the first optical signal are provided to the plurality of first outputs. Each first output may thus receive a fraction of the first optical signal.

The first waveguide system may be configured to provide different path lengths of the first optical signal. Thus, the fractions of the first optical signal being received by different first outputs have traveled along different path lengths. The differences of the path lengths provided by the first waveguide system is further configured in such manner that such that the relation between the path length traveled by the reference beam and the response beam as detected by a light sensor is different for different light sensors.

It should be realized that the path length traveled by the reference beam may differ between different light sensors. Further, the path length traveled by the response beam may also differ between the different light sensors. However, these differences in path lengths of the reference beam and the response beam between different light sensors are not the same, such that a net difference in path length of the reference beam and the response beam differs for different light sensors. In other words, the path length relation, i.e., a difference in path length of the reference beam to the path length of the response beam, is different for different light sensors.

It should be realized that light interacting with the sample travels first as a sample beam to the sample and then as a response beam to the receiving unit. When referring to the path length of the response beam, it should be realized that this implies that path length traveled by the sample beam from the splitter to reach the sample and the path length traveled by the response beam from the sample to the light sensor. When referring to the path length of the reference beam, it should be realized that this implies the path length of the reference beam from the splitter to the light sensor.

It should be realized that a particular path length traveled by the reference beam may correspond to a particular depth into the sample. Thus, for the particular depth into the sample, a path length defined by the response beam may be equal to the path length of the reference beam. Thus, if back-scattering of light occurs at the particular depth, this back-scattered light will be in phase with the reference beam that has traveled the same path length and may be visible in detection of interference between the reference beam and the response beam. Thus, the different path length relations may relate to a relation between the path length of the reference beam and a fixed point on the sample, such as a surface of the sample, such that a light sensor being configured to detect interference of the reference beam and the response beam at a particular path length relation, will detect constructive interference of light from a particular depth in relation to the fixed point on the sample.

The device may be configured such that the plurality of light sensors may detect at least 8 different path length relations between the reference beam and the response beam, such as at least 16 different path length relations, such as at least 64 different path length relations, such as at least 128 different path length relations. The different path length relations may provide a resolution of information of the sample in a direction along the sample beam, i.e., along a depth into the sample. Thus, providing a large number of different path length relations implies that a large resolution of depth information of the sample may be provided.

The one or more waveguides of the first waveguide system may be any structure providing guiding of the first optical signal. The waveguide may be configured to confine light in a cross-section perpendicular to an extension of the waveguide. The waveguide may thus be configured restrict light to follow a path defined by the extension of the waveguide, whereby transmission of light with low loss may be provided. The waveguide may for instance guide light by light being reflected on inner walls of the waveguide by total internal reflection or by a reflective coating being provided on the walls of the waveguide. The waveguide is configured to propagate the first optical signal along the path of the waveguide.

The waveguide may be formed by any material suitable for propagating the light with low loss, i.e. having low absorption in the wavelength range of light. The waveguide may for instance be formed by silicon or silicon nitride. However, it should be realized that other materials may be used instead.

Each light sensor in the plurality of light sensors may be configured to detect an intensity of light. The light sensor may be configured to provide an electrical signal, such as a current, voltage or charge, in dependence of a received intensity of light. By the term "light sensitive area" is here meant an area on a device configured to detect light impinging onto the area, by generating an electrical signal as a response to the light intensity. The light sensor may for instance be formed by a photodiode, a photo-multiplier tube (PMT), or a pixel on an image sensor, such as based on charge-coupled device (CCD) technology or on complementary metal oxide semiconductor (CMOS) technology.

The plurality of light sensors may be arranged in an array of light sensors, such as in a one-dimensional array or a two-dimensional array. It should be realized that the plurality of light sensors may be regularly arranged in the array with equal distances between neighboring light sensors in the array. Thus, an array of light sensors may be utilized, such as an array of light sensors arranged in an image sensor. However, it should be realized that an array of light sensors may be utilized but not all of the light sensors in the array need be used for detecting interference of the reference beam and the response beam. Thus, the plurality of light sensors may be arranged within the array of light sensors without all of the light sensors in the array being used. As yet another alternative, the light sensors of the plurality of light sensors are arranged in any relation to each other and need not be regularly arranged in an array.

The device may be configured to cause interference of the reference beam and the response beam to form a combined signal, which may then be provided to the light sensor. Alternatively, the reference beam and the response beam may both be incident on the light sensor such that interference of the reference beam and the response beam may occur at the light sensor.

The device provides parallel detection of different path length relations between the reference beam and the response beam. This may be achieved with a passive optical set-up such that no active control of optical components of the device is necessary in order to acquire information corresponding to different path length relations.

However, it should further be realized that even though no active control may be necessary, in some embodiments, active control may be used. For instance, fine-tuning of a path length in the at least first waveguide system may be provided by active control, for example by using a phase shifter, such as a thermal phase shifter. This may for instance be used for correcting any errors in manufacturing of desired path lengths in the at least first waveguide system.

According to an embodiment, the first waveguide system comprises a plurality of branches, wherein the first waveguide system is configured to split the first optical signal into a respective split portion for each of the branches, wherein each branch is configured to transport the respective split portion to a unique first output of the plurality of first outputs.

Thus, the first optical signal is split in order to provide parallel detection of different path length relations between the reference beam and the response beam.

The path length provided by the branches may be different for different branches in order to provide the different path length relations between the reference beam and the response beam.

According to an embodiment, the first waveguide system is configured to split the first optical signal such that an optical power of each of the respective split portions is identical.

Thus, the light sensors may receive equal optical power of the fractions of the first optical signal. This implies that different light sensors may acquire information of the sample with equal or at least similar signal-to-noise ratio.

The first waveguide system may be configured to split the first optical signal in a single splitter, such that the first optical signal is split into the plurality of split portions in the same splitter. Alternatively, the first waveguide system may be configured to split the first optical signal in a sequence of splitting the first optical signals into equal halves. As yet another alternative, fractions of the first optical signal may be extracted in a sequence of extractions, wherein a first split portion for a first branch may first be extracted from the first optical signal, followed by a second split portion for a second branch being extracted from the remaining portion of the first optical signal, and so on. In such case, the first waveguide system may be configured to extract an increasing fraction in the sequence from the first optical signal such that the split portions have equal optical power.

According to an embodiment, each first output is associated with a grating coupler for directing the first optical signal transported by the first waveguide system to the light sensor associated with the first output.

Thus, a grating coupler may be used for redirecting light propagated by the first waveguide system towards the light sensor. The first output of the first waveguide system may be connected to the grating coupler for providing the first optical signal to the grating coupler. For instance, the grating coupler may be formed in a waveguide of the first waveguide system at the first output. Alternatively, the first optical signal may be coupled out of the first waveguide system at the first output and the out-coupled first optical signal may further be provided to the grating coupler.

The plurality of light sensors and the first waveguide system may be arranged in different layers. For instance, the first waveguide system may be arranged in a layer above the plurality of light sensors. Each light sensor may receive light to be detected at a surface of the light sensor. The plurality of light sensors may be arranged such that the surface through which light is received by the light sensors faces the layer in which the first waveguide system is arranged.

The grating coupler facilitates directing of the first optical signal out of a plane in which the waveguides of the first waveguide system is arranged. The grating coupler may therefore be useful when the first waveguide system and the plurality of light sensors are arranged in different layers.

According to an embodiment, the first waveguide system is configured to receive the first optical signal being the reference beam, wherein the first waveguide system comprises a common delay element configured to provide a common delay of the reference beam corresponding to a round-trip time of the sample beam being output towards the sample and forming the response beam being received back from the sample.

The sample beam needs to be output to the sample for forming the response beam, which may further be received by the receiving unit. This implies that the sample beam and the response beam need to travel to the sample and back. The reference beam however could be received by the receiving unit directly from the splitter.

Hence, by providing a common delay element, the reference beam may be delayed by a time corresponding to the round-trip time of the sample beam and the response beam to a fixed position at the sample.

The device may be used with a system in which a sample position is accurately known. For instance, if a retina is to be examined, the system may guide a position of the head of the person to be examined such that the retina is placed in an appropriate position.

The common delay element may be common to all of the first outputs. Thus, before the reference beam is split or guided to the different first outputs, the reference beam may be propagated through the common delay element. Thus, differences in the path length of the reference beam as provided by the first waveguide system may be provided after the reference beam has passed the common delay element.

By using the common delay element, the only difference between the path lengths traveled by the reference beam and the response beam may be based on guiding of the reference beam and/or the response beam in waveguide system(s) of the device. This implies that the reference beam and the response beam have been at least substantially equally delayed, providing corresponding relation between the reference beam and the response beam as in a Michelson interferometer.

Since the coherence length of the light signal being split into the reference beam and the sample beam is short, such as in micrometer scale, the common delay element may be needed in order to ensure that the reference beam and the response beam have been correspondingly delayed in order for the reference beam and the response beam to be able to constructively interfere.

According to an embodiment, the device further comprises a second waveguide system configured to receive a second optical signal, wherein the first optical signal is the reference beam and the second optical signal is the response beam, wherein the second waveguide system is configured to transport the second optical signal along different path lengths to a plurality of second outputs; wherein the different light sensors are associated with different second outputs of the plurality of second outputs of the second waveguide system such that a combination of the path length of the reference beam in relation to the path length of the response beam is different for the different light sensors.

As mentioned above, the device may comprise at least a first waveguide system. This implies that the device may comprise a single waveguide system, but the device may alternatively, in addition, comprise the second waveguide system.

Thus, the first and the second waveguide systems may be used, respectively, for guiding the first optical signal being the reference beam and for guiding the second optical signal being the response beam.

It should be realized that, if a single waveguide system is used, the reference beam may be guided through the first waveguide system for providing the reference beam to each of the light sensors. The response beam may travel through free space and may be directly incident on the light sensors without passing through any waveguide system.

According to an alternative, the response beam may be guided through the first waveguide system and the reference beam may be configured to propagate through free space and may be directly incident on the light sensors without passing through any waveguide system.

The use of the first and second waveguide systems ensure that an accurate control is provided of the paths along which each of the reference beam and the response beam travels. Thus, although the use of the first and the second waveguide systems requires the device to be provided with more waveguides, the device may provide a more accurate interferometry detection.

According to an embodiment, the at least the first waveguide system is configured to define a smallest difference between path length relations of the reference beam and the response beam, wherein each light sensor is associated with a path length relation being a multiple of the smallest difference.

The smallest difference between the path length relations may correspond to an axial resolution of the interferometry detection along a direction of the sample beam into the sample. In other words, the smallest difference may provide a depth resolution of the interferometry detection.

Each light sensor being associated with a multiple of the smallest difference implies that an equal axial resolution is used throughout the depth of the sample. However, if some depths are of particular interest, different axial resolutions may be desired along the depth into the sample. In such case, all of the path length relations need not be multiples of the smallest difference.

The device may be configured to acquire information along the direction of the sample beam extending to a range corresponding to a number of different path length relations being used in detection. Thus, the number of different path length relations times the smallest difference (axial resolution) may provide the range of the depth information acquired by the device.

According to an embodiment, the device further comprises a plurality of additional light sensors configured to detect additional information relating to the sample, wherein the additional light sensors and the plurality of light sensors are arranged in an array.

Thus, the device may be configured to detect additional information of the sample which may be combined with the interferometry detection to provide a more complete analysis of the sample.

The device may be configured to utilize a single array of light sensors, such that the additional light sensors and the plurality of light sensors for interferometry detection may be arranged in the same array. Thus, by not using all of the light sensors in the array for interferometry detection, the additional information may be acquired with a compact set-up, using a single array of light sensors for acquiring both information based on the interferometry detection and additional information.

According to a second aspect, there is provided a system for interferometry detection, said system comprising: a splitter configured to receive a light signal and configured to split the light signal into a reference beam and a sample beam, or a receiver unit configured to receive the reference beam and the sample beam formed by splitting the light signal, wherein the system is configured to output the sample beam towards a sample for interacting with the sample beam to form a response beam carrying information of the sample; an array of interferometry detection units configured to detect spatially resolved information of the sample based on each interferometry detection unit being configured to detect information relating to a unique spatial position, wherein each interferometry detection unit comprises: at least a first waveguide system configured to receive a first optical signal, wherein the first optical signal is the reference beam or the response beam, wherein the first waveguide system is configured to transport the first optical signal along different path lengths to a plurality of first outputs; a plurality of light sensors, wherein each light sensor is configured to receive a combination of the reference beam and the response beam forming an interference of the reference beam and the response beam, wherein different light sensors of the plurality of light sensors are associated with different first outputs of the plurality of first outputs of the first waveguide system such that a path length of the reference beam in relation to a path length of the response beam is different for the different light sensors, wherein the plurality of light sensors are configured to simultaneously detect interference of the reference beam and the response beam corresponding to different path length relations.

Effects and features of this second aspect are largely analogous to those described above in connection with the first aspect. Embodiments mentioned in relation to the first aspect are largely compatible with the second aspect.

The system may comprise a plurality of devices according to the first aspect. Thus, each of the interferometry units may be formed by a device according to the first aspect.

The system may comprise a receiver unit which receives the reference beam and the sample beam. The system may comprise a splitter which may be configured to form the reference beam and the sample beam by splitting a light signal. However, the system may receive the reference beam and the sample beam from an external unit.

As yet another alternative, the system may comprise a plurality of splitters. Each splitter may be associated with a respective interferometry detection unit such that separate sample beams may be formed for each of the interferometry detection units.

The system may be configured to provide interferometry detection with a spatial resolution. The different interferometry detection units may thus be related to different spatial positions. This implies that the system may be configured to acquire information of a sample in three dimensions simultaneously, by using the different interferometry detection units for acquiring information across two dimensions in lateral directions of the sample and using each interferometry detection unit for providing depth resolution extending into the sample in a direction perpendicular to the lateral directions and along a direction of the sample beam.

According to an embodiment, the array of interferometry detection units is a two-dimensional array.

It should be realized that the system may be configured to detect information relating to unique spatial positions across two dimensions. This is facilitated by arranging the interferometry detection units in a two-dimensional array.

However, it should be realized that the array of interferometry detection units may alternatively be a one-dimensional array. The system may in such case be configured to detect information relating to unique spatial positions along a line in a lateral direction of the sample.

According to an embodiment, the array of interferometry detection units is integrated on a common substrate.

In addition, the splitter or splitters of the system may also be integrated on the common substrate. The common substrate may be formed by any suitable material. For instance, the substrate may be formed by silicon or silicon oxide (glass).

Thus, the system may be implemented as a photonic integrated circuit. The photonic integrated circuit is an integrated device wherein two or more photonic components form a functioning circuit for transporting and/or processing optical signals. The system may also be referred to as being provided on chip.

It should be further realized that the entire system may be implemented as a single photonic integrated circuit but in other embodiments parts of the system are provided as a photonic integrated circuit. Also, the system may be implemented by a plurality of photonic integrated circuits. For instance, each interferometry detection unit may be formed by a separate photonic integrated circuits. The plurality of photonic integrated circuits may then be mounted on the common substrate.

The system being implemented by integrated parts implies that the system may be very compact.

According to an embodiment, the system is configured to output the sample beam towards biological tissue, such as a retina, for performing Optical Coherence Tomography (OCT).

The system is particularly suited for performing OCT. The system may provide OCT measurements using a compact setting, with no need for bulky components for achieving scanning. The system may further provide OCT measurements in a very fast manner since depth information is provided simultaneously using the different path length relations of the reference beam and the response beam received by the different light sensors.

In OCT measurements, a broadband light source may be used, which may be part of the system or may be external to the system. For instance, for OCT measurement of a retina, a light source having a bandwidth of 70 - 110 nm in a wavelength range of 800 - 1100 nm may be used. For OCT measurement of skin or endoscopy, a light source having a bandwidth of approximately 100 nm in a wavelength range of 1200 - 1400 nm may be used.

According to an embodiment, the system is integrated with a scanning laser ophthalmoscopy (SLO) module configured to detect a reflectance and/or fluorescence image of the biological tissue, such as the retina.

This implies that the system is configured to detect additional information of the biological tissue based on both interferometry detection, such as in an OCT measurement, and on reflectance and/or fluorescence imaging, such as performed by the SLO module. This enables improved analysis of the biological tissue within a compact system.

The SLO module may comprise light sensors arranged in an array such that the SLO module may be configured to detect spatially resolved information from the biological tissue.

The SLO module may utilize one or more additional light sensors configured to detect additional information, wherein the additional light sensors are arranged in a common array with the light sensors of the array of interferometry detection units.

According to another embodiment, the system is integrated with a Hyperspectral Imaging (HSI) module configured to capture and process information across multiple wavelength bands.

This implies that the system is configured to detect additional information of the biological tissue based on both interferometry detection, such as in an OCT measurement, and on hyperspectral imaging, such as performed by the HSI module. The system may further be configured to detect additional information using reflectance and/or fluorescence imaging, such as performed by the SLO module. This enables improved analysis of the biological tissue within a compact system.

The HSI module may be configured to capture information across multiple wavelength bands, such as at least four, such as at least 16, such as at least 32 wavelength bands. Each wavelength band may be narrow, such as corresponding to a range of wavelengths having a width of less than 50 nm, such as less than 20 nm, determined by a full width at half maximum of a peak of sensitivity of the wavelength band. The HSI module may be configured to detect light in a contiguous spectral range. However, the HSI module may alternatively be configured to detect light in a plurality of discrete wavelength bands.

The HSI module may be configured to detect light in a wavelength range within an ultraviolet light part of the electromagnetic spectrum, a visible light part of the electromagnetic spectrum and/or an infrared light part of the electromagnetic spectrum.

The HSI module may comprise light sensors arranged in an array such that the HSI module may be configured to detect spatially resolved information from the biological tissue.

The HSI module may utilize one or more additional light sensors configured to detect additional information, wherein the additional light sensors are arranged in a common array with the light sensors of the array of interferometry detection units.

According to a third aspect, there is provided a method for interferometry detection, said method comprising: receiving a reference beam and a response beam, wherein the response beam is a response to a sample beam formed by splitting a light signal into the reference beam and the sample beam and wherein the sample beam is directed towards a sample for interacting with the sample beam to form the response beam carrying information of the sample; receiving, by a first waveguide system, a first optical signal, wherein the first optical signal is the reference beam or the response beam, transporting, by the first waveguide system, the first optical signal along different path lengths to a plurality of first outputs; receiving, by a plurality of light sensors, a combination of the reference beam and the response beam forming an interference of the reference beam and the response beam, wherein a path length of the reference beam in relation to a path length of the response beam is different for different light sensors; and simultaneously detecting, by the plurality of light sensors, interference of the reference beam and the response beam corresponding to different path length relations.

Effects and features of this third aspect are largely analogous to those described above in connection with the first and second aspects. Embodiments mentioned in relation to the first and second aspects are largely compatible with the third aspect.

This implies that the method enables simultaneous detection of different path length relations between the reference beam and the response beam. Hence, instead of scanning a mirror in a path of the reference beam, the different path length relations may be provided by at least the transporting of the first optical signal along different path lengths.

Brief description of the drawings

The above, as well as additional objects, features, and advantages of the present description, will be better understood through the following illustrative and non-limiting detailed description, with reference to the appended drawings. In the drawings like reference numerals will be used for like elements unless stated otherwise.

Fig. 1 is a schematic view of a conventional set-up for interferometry detection.
Fig. 2 is a schematic top view of a device for interferometry detection according to a first embodiment.
Fig. 3a is a schematic top view of a device for interferometry detection according to a second embodiment.
Fig. 3b is a schematic cross-sectional view of the device of Fig. 3a taken along line A-A in Fig. 3a.
Fig. 4 is a schematic view of a system according to an embodiment.
Fig. 5 is a flow chart of a method according to an embodiment.

Detailed description

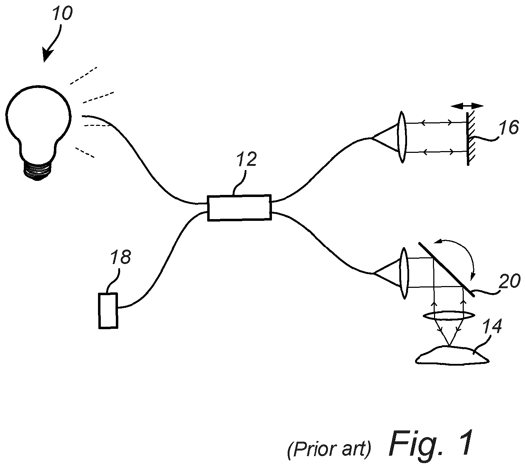

Referring now to Fig. 1, a typical set-up for interferometry detection according to prior art will be discussed. This set-up may be used for instance in optical coherence tomography (OCT).

A light source 10 generating light with low coherence length (in micrometer scale) is provided. The light is provided to a beam splitter 12 which splits light into a sample beam and a reference beam. The sample beam is directed towards a sample 14 and may be focused onto a spot on the sample 14. Light that is reflected or back-scattered directly back from the sample 14 will form a response beam that travels back towards the beam splitter 12. The reference beam travels along a reference path to a mirror 16 and is reflected back from the mirror 16 towards the beam splitter 12.

The reference beam and the response beam may thus be re-combined at the beam splitter 12 and the re-combined light may be directed towards a photodetector 18. The reference beam and the response beam may thus interfere such that interferometry detection is provided at the photodetector 18.

The interferometry detection allows recording of photons in the response beam that has traveled an equal path length to the reference beam to be detected. It should be noted that the sample 14 may scatter light into a large range of angles and that diffusely scattered light (experiencing multiple scattering events) may provide background light towards the beam splitter 12. However, thanks to the interferometry detection, constructive interference of light that has traveled equal path lengths in a sample path towards the sample 14 and back and in a reference path towards the mirror 16 and back will occur, allowing rejection of most photons that scatter multiple times.

By providing an axial scanning of the mirror 16 along a direction of propagation of the reference beam, different path lengths of the reference path will be provided in order to allow detection of light having traveled different path lengths along the sample path. This allows acquiring information from different depths into the sample 14. For instance, using OCT for analysis of a biological sample, depth information into tissue of the biological sample may be acquired.

Depth information may typically be acquired up to a range of 1-2 mm into the tissue. At greater depths, a proportion of light being back-scattered without experiencing more than one scattering event may be too small for detection.

The sample beam may be directed to the sample via a scanning mirror 20. This mirror may be tilted for providing lateral scanning of a position of the spot of the sample beam on the sample 14. Thus, scanning of the spot in two lateral directions may be provided.

The tilting of the scanning mirror 20 and the axial scanning of the mirror 16 may hence enable imaging of the sample in three dimensions.

In particular, the axial scanning of the mirror 16 may be referred to time-domain (TD) OCT, as different depth information of the sample may be acquired at different time points based on the scanning of the position of the mirror 20.

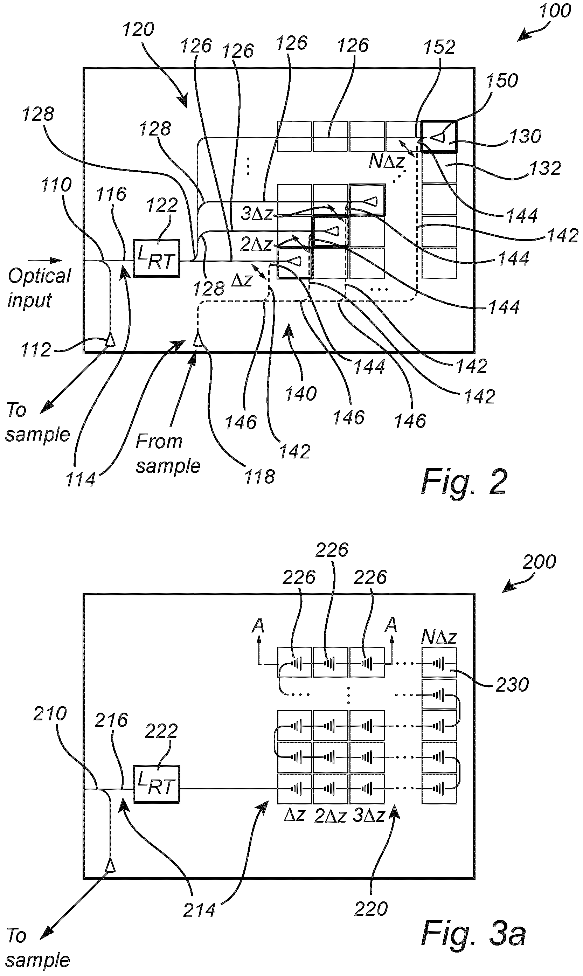

Referring now to Fig. 2, a device 100 for interferometry detection according to a first embodiment will be described. The device 100 for interferometry detection may be configured to acquire similar information as described above in relation to Fig. 1. However, the device 100 may perform interferometry detection without necessarily including any scanning of mirrors. Thus, the device 100 may be very compact as no bulky optical components are needed.

The device 100 may comprise a splitter 110 configured to receive a light signal and configured to split the light signal into a reference beam and a sample beam. The reference beam and the sample beam may be split by the splitter 110 into separate waveguides that may be configured for propagating the reference beam and the sample beam, respectively. The splitter 110 may for instance be implemented by a directional coupler.

The device 100 may further be configured to output the sample beam towards a sample. The device 100 may for instance comprise an output grating coupler 112 configured to output the sample beam from the waveguide towards the sample. The output grating coupler 112 may thus be directed towards a defined position in which a sample to be analyzed is to be arranged.

The light signal may be generated by a light source which may be external to the device. The light source may for instance be a broadband light source that may generate light having a short coherence length.

The splitter 110 may also be external to the device 100 and the output of the sample beam towards the sample may also take place external to the device 100.

The device 100 may instead comprise a receiving unit 114 for receiving the reference beam and a response beam, wherein the response beam is a response formed by the sample interacting with the sample beam. The response beam may thus carry information of the sample.

The receiving unit 114 may thus comprise a waveguide 116, which is configured to receive the reference beam from the splitter 110 (being part of the device 100 or being external to the device 100). The receiving unit 114 may further be configured to receive the response beam. As shown in Fig. 2, the receiving unit 114 may comprise an input grating coupler 118 configured to receive the response beam from the sample and to direct the response beam into a waveguide.

The device 100 comprises at least a first waveguide system 120 configured to receive a first optical signal. As shown in Fig. 2, the first optical signal received by the first waveguide system 120 is the reference beam. However, it should be realized that the first waveguide system may alternatively be configured to receive the response beam. Hereinafter, the first waveguide system 120 will be described as being configured to transport the reference beam but it should be realized that in some embodiments the first waveguide system may instead be configured to transport the response beam.

The first waveguide system 120 is configured to transport the reference beam along different path lengths. The first waveguide system 120 may comprise a plurality of branches 124. The first waveguide system 124 is configured to split the reference beam into a respective split portion for each of the branches 124, wherein each branch 124 is configured to transport the respective split portion to a unique first output of a plurality of first outputs 126.

The first waveguide system 124 may comprise a sequence of directional couplers 128, such that each directional coupler 128 is configured to split off a split portion of the reference beam to a branch 124, wherein the remaining portion of the reference beam is transported in a waveguide towards a following directional coupler 128 in the sequence.

The first waveguide system 120 may be configured to split the reference beam such that an optical power of each of the respective split portions is identical. Thus, the optical power of the reference beam in each of the branches 124 may be identical. This may be achieved for instance by extracting different fractions of the reference beam in the sequence of directional couplers 128, such that a first directional coupler may extract a first fraction of the reference beam, whereas a second directional coupler may extract a second fraction of the remaining portion of the reference beam, and so on. The second fraction may be larger than the first fraction in order for the optical power of each of the split portions to be identical.

The first waveguide system 120 is configured to transport the reference beam along different path lengths to the plurality of first outputs 126. Thus, the branches 124 may comprise waveguides of differing lengths to define the different path lengths.

The device 100 may further comprise a second waveguide system 140 configured to receive a second optical signal. As shown in Fig. 2, the second optical signal received by the second waveguide system 140 is the response beam. However, it should be realized that the second waveguide system may alternatively be configured to receive the reference beam. Hereinafter, the second waveguide system 140 will be described as being configured to transport the response beam but it should be realized that in some embodiments the second waveguide system may instead be configured to transport the reference beam.

The second waveguide system 140 is configured to transport the response beam along different path lengths. The second waveguide system 140 may comprise a plurality of branches 142. The second waveguide system 142 is configured to split the response beam into a respective split portion for each of the branches 142, wherein each branch is configured to transport the respective split portion to a unique second output of a plurality of second outputs 144.

The second waveguide system 140 may comprise a sequence of directional couplers 146, such that each directional coupler 146 is configured to split off a split portion of the response beam to a branch 142, wherein the remaining portion of the response beam is transported in a waveguide towards a following directional coupler 146 in the sequence.

The second waveguide system 140 may be configured to split the response beam such that an optical power of each of the respective split portions is identical. Thus, the optical power of the response beam in each of the branches 142 may be identical. This may be achieved for instance by extracting different fractions of the response beam in the sequence of directional couplers 146, such that a first directional coupler may extract a first fraction of the response beam, whereas a second directional coupler may extract a second fraction of the remaining portion of the response beam, and so on. The second fraction may be larger than the first fraction in order for the optical power of each of the split portions to be identical.

The second waveguide system 140 is configured to transport the response beam along different path lengths to the plurality of second outputs 144. Thus, the branches 142 may comprise waveguides of differing lengths to define the different path lengths.

The device 100 further comprises a plurality of light sensors 130. Each light sensor 130 is configured to receive a combination of the reference beam and the response beam, wherein the combination of the reference beam and the response beam forms an interference of the reference beam and the response beam.

Different light sensors 130 are associated with different first outputs 126 of the first waveguide system 120 and different second outputs 144 of the second waveguide system 140. The difference in path length traveled by the reference beam and the response beam based on response from a fixed axial location at the sample is referred to as a path length relation between the reference beam and the response beam. This path length relation is set by the path lengths of the branches 124 and the branches 142. Different light sensors 130 are configured to receive a combination of the reference beam and the response beam, such that that a path length of the reference beam in relation to a path length of the response beam is different for the different light sensors 130, i.e., the path length relations are different.

The sample beam being directed to the fixed axial location at the sample will cause a response beam which carries information relating to different sample path lengths depending on an axial location, i.e., along a direction of propagation of the sample beam, of a back-scattering event in relation to the fixed axial location at the sample. The different light sensors 130 receiving a combination of the reference beam and the response beam with different path length relations will thus be configured to detect constructive interference of light for different axial locations of the back-scattering event. Hence, different light sensors 130 may be configured to detect different depth information of the sample.

Thanks to the device 100 being configured to split the reference beam and the response beam such that different light sensors 130 will receive different portions of the combination of the reference beam and the response beam, the plurality of light sensors 130 are configured to simultaneously detect interference of the reference beam and the response beam for the different path length relations. Thus, the device 100 is configured to acquire depth information of the sample using interferometry detection without a need for an axial scanning of a mirror for controlling a path length of the reference path.

It should be realized that the combination of the different path lengths of the branches 124 of the first waveguide system 120 and the different path lengths of the branches 142 of the second waveguide system 140 may be formed such that different path length relations are provided to the plurality of light sensors 130. Hence, the path lengths of the branches 142 may be different for different branches 142 and the path lengths of the branches 124 may be different for the different branches 124. However, the relations between path lengths of the branches 142 may not be the same as the relations between path lengths of the branches 124.

Thus, the different light sensors 130 are associated with different second outputs of the plurality of second outputs 144 of the second waveguide system 140 such that a combination of the path length of the reference beam in relation to the path length of the response beam is different for the different light sensors 130.

A first output 124 of the first waveguide system 120 and a second output 144 of the second waveguide system 140 may be connected to a coupling unit 128 for combining the split portion of the reference beam and the split portion of the response beam from the respective first output 124 and the second output 144. The coupling unit 128 may output a combination of the split portion of the reference beam and the split portion of the response beam to a waveguide 152 based on interference of the reference beam and the response beam. The coupling unit 128 may for instance be formed by a 2x2 multimode interferometer (MMI), wherein constructive interference is guided a first output of the coupling unit 128 connected to the waveguide 152, whereas a second output of the coupling unit 128 may be connected to a terminator, wherein the light signal is absorbed to avoid any unwanted back reflections.

The waveguide 152 may further propagate the combination of the reference beam and the response beam to a grating coupler 150 for directing the interference signal based on the reference beam and the response beam to the light sensor 130.

The grating coupler 150 may be configured to direct the signal from the waveguide 152 out of a plane in which the waveguide 152 is arranged and towards a plane in which the plurality of light sensors 130 are arranged. Thus, the plurality of light sensors 130 may be arranged at a surface of a substrate. The first and second waveguide systems 120, 140 may be arranged in a layer above the surface of the substrate. This may facilitate guiding of the reference beam and the response beam for providing the reference beam and the response beam to the plurality of light sensors 130.

The first waveguide system 120 may comprise a common delay element 122 configured to provide a common delay of the reference beam corresponding to a round-trip time of the sample beam being output towards the sample and forming the response beam being received back from the sample. Thus, the common delay element 122 is common to the portion of the reference beam being received by each of the first outputs 126. The reference beam may thus be propagated through the common delay element 122 before being split into different branches 124.

The common delay element 122 may be achieved by a waveguide that provides a path length for delaying the reference beam. This may for instance be achieved by forming a waveguide in a spiral path so as to achieve a long delay in a small footprint of the device 100. The common delay element 122 may also or alternatively include a material having a high refractive index causing a delay of the reference beam. According to yet another alternative, the common delay element 122 may be actively controlled, for instance by using a phase shifter, such as a thermal phase shifter.

The path length relations of the reference beam and the response beam for the light provided to the respective light sensors 130 may be controlled by the path lengths of the branches 124 and the branches 142 with the common delay element 122 of the first waveguide system 120 ensuring that the reference beam is subject to a corresponding path length to the roundtrip of the sample beam and the response beam. The device 100 may be designed to create a gradual increase in an optical path length difference (OPLD) between the reference beam and the response beam.

The OPLD for the light sensors 130 can follow a patten such that the OPLD is a multiple of a smallest difference $\Delta z$ in the OPLD. Hence, the light sensors 130 may be configured to receive light based on interference between the reference beam and the response beam, wherein the OPLD is $\Delta z$, $2\Delta z$, $3\Delta z$, ..., $N\Delta z$. The smallest difference thus provides an axial resolution of depth information in the sample and $N\Delta z$ corresponds to a range of the depth information. The device 100 may thus comprise $N$ light sensors 130 such that each light sensor 130 is associated with a unique path length relation.

As shown in Fig. 2, the device 100 may comprise a plurality of additional light sensors 132 that may be arranged in a common array with the plurality of light sensors 130. The plurality of light sensors 130 and additional light sensors 132 may thus be formed by an image sensor, such as a charge-coupled device (CCD) or a complementary metal oxide semiconductor (CMOS) image sensor.

The additional light sensors 132 may be used for acquiring additional information relating to the sample. This may be used for providing a multi-modality of the device 100 such that the device 100 may be selectively configured to read out information from the light sensors 130 or from the additional light sensors 132. Alternatively, the device 100 may be configured to read out information from both the light sensors 130 and the additional light sensors 132 in order to provide multiple types of information from the sample.

For instance, if the device 100 is used for performing OCT on an eye, the additional light sensors 132 may be used as fundus imaging detectors for forming an image of the fundus of the eye. The additional light sensors 132 may be configured to acquire information in a plurality of narrow wavelength bands, so as to provide a hyperspectral image of the fundus.

The additional light sensors 132 may also or alternatively comprise additional light sensors be configured for detecting a reflectance and/or fluorescence image of the sample. Thus, the additional light sensors 132 may be used for scanning laser ophthalmoscopy (SLO) imaging.

The output grating coupler 112 and the input grating coupler 118 of the device 100 may function like a detector pinhole. This implies that the interferometry detection by the device 100 may be performed using confocal imaging. This helps to reduce crosstalk by rejecting out-of-focus light allowing the device 100 to perform interferometry detection with a high signal-to-noise ratio.

The output grating coupler 112 and the input grating couple 118 may be combined into a single unit using a directional coupler or an optical circulator.

It should be realized that the device 100 need not comprise any additional light sensors 132. The plurality of light sensors 130 may be provided in any arrangement and need not be arranged in a two-dimensional array. For instance, the plurality of light sensors 130 may be arranged in a one-dimensional array or in a line or column in a two-dimensional array (where multiple devices are arranged side-by-side). Further, the plurality of light sensors 130 may be provided in an arbitrary arrangement with any relation between the light sensors 130, not necessarily being arranged in a regular array.

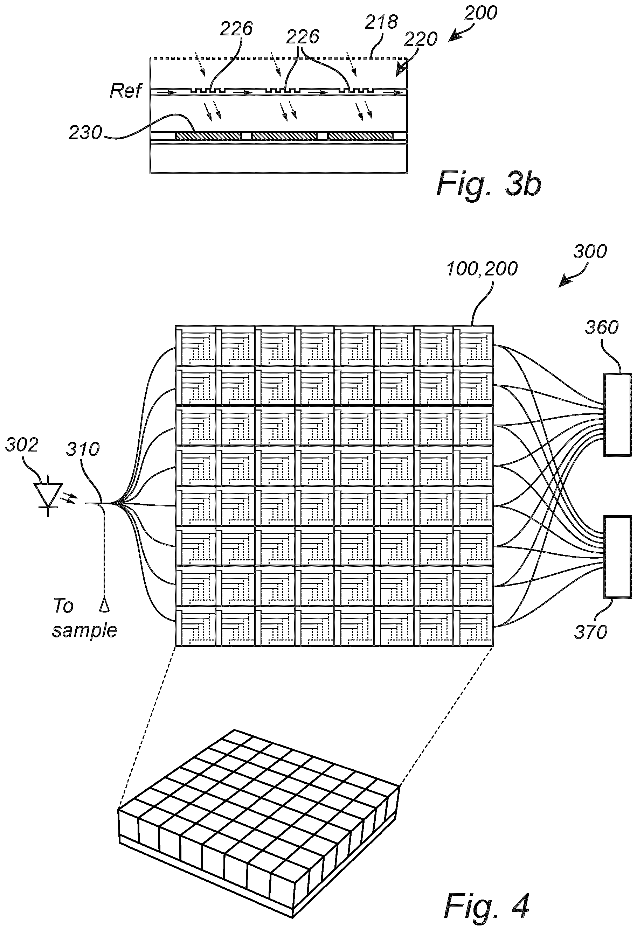

Referring now to Figs 3a-b, a device 200 according to a second embodiment will be described. The device 200 is similar to the device 100 of the first embodiment and for brevity, only differences between the embodiments will be discussed below. Fig. 3a shows a top view of the device 200 and Fig. 3b shows a cross-sectional view of a portion of the device 200 taken at line A-A in Fig. 3a.

The device 200 only comprises a single waveguide system 220 configured to receive the reference beam. The response beam is guided to be received directly onto the light sensors 230. Hereinafter, the waveguide system 220 will be described as being configured to transport the reference beam. However, it should be realized that the reference beam may be output to an external mirror and may be incident directly onto the light sensors, whereas the response beam may be received into the single waveguide system and be guided by the waveguide system to transport the response beam to the respective light sensors.

The waveguide system 220 may be configured to transport the reference beam in a layer arranged above a substrate in which the plurality of light sensors 230 are arranged. The waveguide system 220 may be configured to pass a common delay element 222 and thereafter transport the reference beam to a sequence of outputs 226. Each output 226 may be formed by a grating coupler for directing the reference beam transported by the waveguide system 220 to the light sensor 230 associated with the output 226.

The grating couplers may be configured to output a fraction of the reference beam and to pass a remaining fraction along the waveguide system 220. The sequence of grating couplers may be configured to output different fractions of the signal received by the respective grating couplers. Thus, a first grating coupler may extract a first fraction of the reference beam, whereas a second grating coupler may extract a second fraction of the remaining portion of the reference beam, and so on. The second fraction may be larger than the first fraction. Thus, the waveguide system 220 is configured to split the reference beam such that an optical power of each of the respective split portions received by different light sensors 230 is identical.

The response beam may be incident on a surface 218 above a layer in which the waveguide system 220 is arranged. Thus, the response beam may be configured to pass through the layer in which the waveguide system 220 is arranged and be incident on the light sensors 230.

The reference beam may have a polarization, whereas the response beam may include further polarization components. Thus, only part of the response beam having a same polarization as the reference beam may contribute to forming interference with the reference beam. Thus, the surface 218 may comprise a polarizer for only passing part of the response beam having the same polarization as the reference beam. Thus, a polarization component which may not contribute to interference and thus to interferometry detection may not be allowed to reach the light sensor 230.

The device 200 may comprise a receiving unit 214 for receiving the reference beam and the response beam. The receiving unit 214 may thus comprise a waveguide 216, which is configured to receive the reference beam from the splitter 210 (being part of the device 200 or being external to the device 200). The receiving unit 214 may further be configured to receive the response beam by receiving the response beam being incident on the surface 218.

The light sensors 230 may be configured to receive a combination of the reference beam and the response beam by the reference beam being received from the output 226 associated with the light sensor 230 and by the response beam being incident onto the surface 218 to reach the light sensor 230. Thus, an interference of the reference beam and the response beam may be formed on the light sensor 230 for allowing interferometry detection by the device 200.

Each of the light sensors 230 are configured to receive the response beam from the response beam being incident onto the surface 218. Thus, the path length traveled by the response beam is the same for each of the light sensors 230. Thus, different path length relations of the reference beam and the response beam are defined by the different path lengths traveled by the reference beam in the waveguide system 220.

The waveguide system 220 may be configured such that a distance between neighboring outputs 226 in the sequence is equal throughout the sequence. Thus, a smallest difference between path length relations is defined by the distance between neighboring outputs 226. This smallest distance may be set to $\Delta z$. The light sensors 230 may thus be configured to detect OPLDs corresponding to $\Delta z$, $2\Delta z$, $3\Delta z$, ..., $N\Delta z$, where $N$ is the number of light sensors 230 of the device 200.

A pixel pitch of an array of pixels in an image sensor may for instance be 2.5 $\mu$m. Thus, if such an image sensor is used for providing the light sensors 230, the waveguide system 220 may be configured to provide a smallest difference $\Delta z = 2.5$ $\mu$m. If the device 200 is used for detecting information of a retina using a wavelength of light of 950 nm and a waveguide system 220 formed in silicon nitride, the refractive index of the waveguide $n_{wg}$ may be 1.68, whereas the refractive index of the retina $n_{retina}$ is 1.38.

This implies that axial resolution of the device 200 may be:

$$\Delta z \cdot \frac{n_{wg}}{n_{retina}} \approx 3 \ \mu m.$$

Further, using 800 light sensors 230, the device 200 may be configured to provide a range of depth information of the sample corresponding to 2.4 mm, which exceeds a typical range of OCT systems.

Referring now to Fig. 4, a system 300 for interferometry detection is shown.

The system 300 may comprise a light source 302 for forming a light signal. The system 300 further comprises a splitter 310 configured to receive the light signal and to split the light signal into a reference beam and a sample beam. The system 300 may alternatively be configured to receive the reference beam and the sample beam from an external splitter.

The system 300 comprises an array of interferometry detection units, wherein each interferometry detection unit may be formed by a device 100 according to the first embodiment or a device 200 according to the second embodiment.

The system 300 may comprise a single light source 302 which provides a light signal for each of the interferometry detection units 100, 200. The light signal from the light source 302 may be split a number of times, such as in a splitting tree arrangement. Thus, a portion of the light signal may be provided to each of the interferometry detection units 100, 200. Each interferometry detection unit 100, 200 may further comprise a splitter 110, 210, as shown in Figs 2-3, for splitting the received portion of the light signal into the reference beam and the sample beam used by the interferometry detection unit 100, 200. Alternatively, the splitting of the light signal into the reference beam and the sample beam may be provided externally to the interferometry detection units 100, 200 and may be provided in the shared splitter 310 of the system 300.

The light signal provided by the light source 302 may be heavily attenuated by splitting the light signal for each of the interferometry detection units 100, 200. The system 300 may further comprise optical amplifiers for amplifying the light signal.

Each interferometry detection unit 100, 200 may be configured to receive the reference beam and the response beam as discussed above.

Thanks to using an array of interferometry detection units 100, 200, a spatial resolution in acquiring information from the sample is provided. The system 300 may be configured to output a plurality of sample beams to form a pattern of spots arranged on the sample. Each interferometry detection unit 100, 200 may be configured to perform interferometry detection for a single spot on the sample for providing depth information along a direction into the sample for the particular spot.

The array of interferometry detection units 100, 200 may be a two-dimensional array. This implies that spectral resolution in two dimensions of the sample may be provided simultaneously.

The system 300 may thus be configured to provide full-field OCT measurements simultaneously acquiring information of a range of depths into the sample. Further, the system 300 may enable confocal imaging such that crosstalk is reduced between different positions on the sample.

However, it should be realized that the interferometry detection units 100, 200 may alternatively be arranged in a one-dimensional array. This may for instance be used for providing imaging of a line of the sample. The imaged line may then be scanned for providing two-dimensional imaging of the sample. Use of a one-dimensional array may facilitate routing of the light signal from the single light source 302 to each of the interferometry detection units 100, 200.

The system 300 may be provided in a compact form-factor. In particular, each device 100, 200 described above may be integrated on a substrate. Further, the array of interferometry detection units 100, 200 may be integrated on a common substrate. Also, the light source 302 and the splitter 310 of the system 300 may be integrated on the common substrate. Thus, the system 300 may be formed by a photonic integrated circuit (PIC), which may be configured to output sample beam(s) towards a sample. The system 300 may further be arranged in an apparatus that does not require any movable optical parts for scanning of the sample.

The system 300 may be configured to output the sample beam towards biological tissue, such as a retina, for performing OCT measurement of the retina.

The system 300 may further be integrated with a scanning laser ophthalmoscopy (SLO) module 360 configured to detect a reflectance and/or fluorescence image of the biological tissue, such as the retina. The SLO module 360 may be configured to output light towards the retina for performing SLO measurement.

The SLO module 360 may further be configured to receive detected information from the additional light sensors 132 of the device 100 such that a single array of light sensors may be used for acquiring information for the OCT measurement and the SLO measurement.

The system 300 may further be integrated with a Hyperspectral Imaging (HSI) module 370 configured to capture and process information across multiple wavelength bands.

The HSI module may be configured to receive detected information from the additional light sensors 132 of the device 100 such that a single array of light sensors may be used for acquiring information for the OCT measurement and the HSI measurement and, possibly, further acquiring information for the SLO measurement.

The additional light sensors 132 may be provided with different light filters, which may be integrated on the respective additional light sensors 132. The light filters may control a wavelength band to be detected by the respective additional light sensors 132. At least four different light filters may be used for providing spectral resolution of imaging of the sample.

OCT measurement may advantageously be combined with SLO measurement and/or HSI measurement for analysis of a retina. Thus, thanks to the system 300 being able to integrate all these measurements into a single apparatus, the system 300 may be adapted for allowing thorough analysis of the retina.

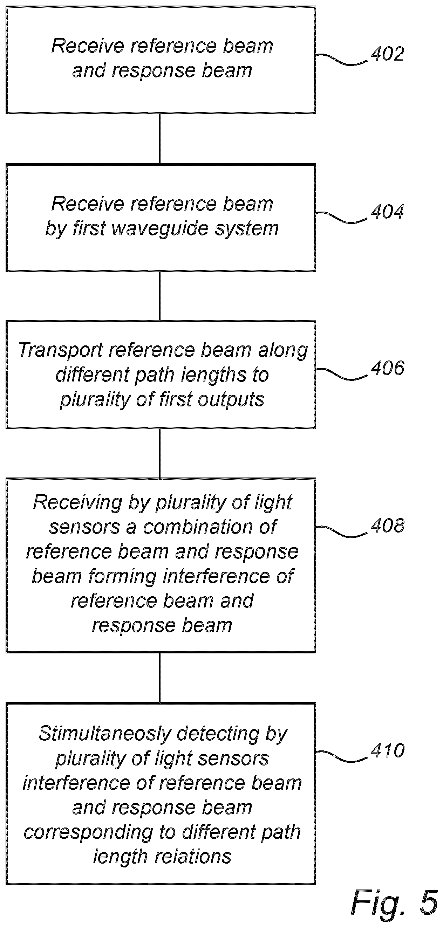

Referring now to Fig. 5, a method for interferometry detection will be described. The method may be performed by any of the devices 100, 200 described above.

The method comprises receiving 402 a reference beam and a response beam. The response beam is a response to a sample beam formed by splitting a light signal into the reference beam and the sample beam and wherein the sample beam is directed towards a sample for interacting with the sample beam to form the response beam carrying information of the sample. The method may also comprise splitting of a light signal to form the reference beam and the sample beam.

The method further comprises receiving 404, by a first waveguide system, a first optical signal. The first optical signal may be the reference beam but may alternatively be the response beam. Hereinafter, the first waveguide system may be referred to as transporting the reference beam.

The method further comprises transporting 406, by the first waveguide system, the reference beam along different path lengths to a plurality of first outputs.

The reference beam may be transported to a sequence of first outputs such that a fraction of the reference beam is split off at each output while a remaining fraction of the reference beam is propagated towards a following output in the sequence. Thus, the different path lengths may be provided based on the different distances along a waveguide passing the sequence of outputs that is traveled by the reference beam.

Alternatively, the reference beam may be split into a plurality of branches, each connected to a respective first output. The branches may have different path lengths in order for the reference beam to be transported along different path lengths to the first outputs.

The method further comprises receiving 408, by a plurality of light sensors, a combination of the reference beam and the response beam forming an interference of the reference beam and the response beam. The reference beam and the response beam may be combined before reaching the light sensors or may be combined at the light sensors. A path length traveled by the reference beam in relation to a path length traveled by the response beam for a fixed axial position on the sample is different for different light sensors. This implies that different light sensors may receive information relating to different axial positions in relation to the fixed axial position.

The method further comprises simultaneously detecting 410, by the plurality of light sensors, interference of the reference beam and the response beam corresponding to different path length relations. Hence, the light sensors may be configured to detect depth information of the sample at a single point in time. Thus, the method allows detecting depth information of a sample in a very fast manner.

In the above the inventive concept has mainly been described with reference to a limited number of examples. However, as is readily appreciated by a person skilled in the art, other examples than the ones disclosed above are equally possible within the scope of the inventive concept, as defined by the appended claims.

**Claims**

1. A device (100; 200) for interferometry detection, said device comprising:

    a receiving unit (114; 214) configured to receive a reference beam and a response beam, wherein the response beam is a response to a sample beam formed by splitting a light signal into the reference beam and the sample beam, wherein the sample beam is directed towards a sample for interacting with the sample beam to form the response beam carrying information of the sample;
    at least a first waveguide system (120; 220) configured to receive a first optical signal, wherein the first optical signal is the reference beam or the response beam, wherein the first waveguide system (120; 220) is configured to transport the first optical signal along different path lengths to a plurality of first outputs (126; 226);
    a plurality of light sensors (130; 230), wherein each light sensor (130; 230) is configured to receive a combination of the reference beam and the response beam forming an interference of the reference beam and the response beam, wherein different light sensors of the plurality of light sensors (130; 230) are associated with different first outputs of the plurality of first outputs (126; 226) of the first waveguide system (120; 220) such that a path length of the reference beam in relation to a path length of the response beam is different for the different light sensors, wherein the plurality of light sensors (130; 230) are configured to simultaneously detect interference of the reference beam and the response beam corresponding to different path length relations.

2. The device according to claim 1, wherein the first waveguide system (120) comprises a plurality of branches (124), wherein the first waveguide system (120) is configured to split the first optical signal into a respective split portion for each of the branches (124), wherein each branch (124) is configured to transport the respective split portion to a unique first output of the plurality of first outputs (126).

3. The device according to claim 2, wherein the first waveguide system (120) is configured to split the first optical signal such that an optical power of each of the respective split portions is identical.

4. The device according to any one of the preceding claims, wherein each first output (126; 226) is associated with a grating coupler for directing the first optical signal transported by the first waveguide system (120; 220) to the light sensor (130; 230) associated with the first output (126; 226).

5. The device according to any one of the preceding claims, wherein the first waveguide system (120; 220) is configured to receive the first optical signal being the reference beam, wherein the first waveguide system (120; 220) comprises a common delay element (122; 222) configured to provide a common delay of the reference beam corresponding to a round-trip time of the sample beam being output towards the sample and forming the response beam being received back from the sample.

6. The device according to any one of the preceding claims, wherein the device (100) further comprises a second waveguide system (140) configured to receive a second optical signal, wherein the first optical signal is the reference beam and the second optical signal is the response beam, wherein the second waveguide system (140) is configured to transport the second optical signal along different path lengths to a plurality of second outputs (144); wherein the different light sensors (130) are associated with different second outputs of the plurality of second outputs (144) of the second waveguide system (140) such that a combination of the path length of the reference beam in relation to the path length of the response beam is different for the different light sensors.

7. The device according to any one of the preceding claims, wherein the at least the first waveguide system (120; 220) is configured to define a smallest difference between path length relations of the reference beam and the response beam, wherein each light sensor (130; 230) is associated with a path length relation being a multiple of the smallest difference.

8. The device according to any one of the preceding claims, further comprising a plurality of additional light sensors (132) configured to detect additional information relating to the sample, wherein the additional light sensors (132) and the plurality of light sensors (130) are arranged in an array.

9. A system (300) for interferometry detection, said system (300) comprising:

a splitter (310) configured to receive a light signal and configured to split the light signal into receive a reference beam and a sample beam, or a receiver unit configured to receive the reference beam and the sample beam formed by splitting the light signal, wherein the system (300) is configured to output the sample beam towards a sample for interacting with the sample beam to form a response beam carrying information of the sample;
an array of interferometry detection units (100; 200) configured to detect spatially resolved information of the sample based on each interferometry detection unit (100; 200) being configured to detect information relating to a unique spatial position,
wherein each interferometry detection unit (100; 200) comprises:

at least a first waveguide system (120; 220) configured to receive a first optical signal, wherein the first optical signal is the reference beam or the response beam, wherein the first waveguide system (120; 220) is configured to transport the first optical signal along different path lengths to a plurality of first outputs (126; 226);
a plurality of light sensors (130; 230), wherein each light sensor (130; 230) is configured to receive a combination of the reference beam and the response beam forming an interference of the reference beam and the response beam, wherein different light sensors of the plurality of light sensors (130; 230) are associated with different first outputs of the plurality of first outputs (126; 226) of the first waveguide system (120; 220) such that a path length of the reference beam in relation to a path length of the response beam is different for the different light sensors (130; 230), wherein the plurality of light sensors (130; 230) are configured to simultaneously detect interference of the reference beam and the response beam corresponding to different path length relations.

10. The system according to claim 9, wherein the array of interferometry detection units (100; 200) is a two-dimensional array.

11. The system according to claim 9 or 10, wherein the array of interferometry detection units (100; 200) are integrated on a common substrate.

12. The system according to any one of claims 9-11, wherein the system (300) is configured to output the sample beam towards biological tissue, such as a retina, for performing Optical Coherence Tomography, OCT.

13. The system according to claim 12, wherein the system (300) is integrated with a scanning laser ophthalmoscopy, SLO, module (360) configured to detect a reflectance and/or fluorescence image of the biological tissue, such as the retina.

14. The system according to claim 12 or 13, wherein the system (300) is integrated with a Hyperspectral Imaging, HSI, module (370) configured to capture and process information across multiple wavelength bands.

15. A method for interferometry detection, said method comprising:

receiving (402) a reference beam and a response beam, wherein the response beam is a response to a sample beam formed by splitting a light signal into the reference beam and the sample beam and wherein the sample beam is directed towards a sample for interacting with the sample beam to form the response beam carrying information of the sample;
receiving (404), by a first waveguide system, a first optical signal, wherein the first optical signal is the reference beam or the response beam,
transporting (406), by the first waveguide system, the first optical signal along different path lengths to a plurality of first outputs;
receiving (408), by a plurality of light sensors, a combination of the reference beam and the response beam forming an interference of the reference beam and the response beam, wherein a path length of the reference beam in relation to a path length of the response beam is different for different light sensors; and

simultaneously detecting (410), by the plurality of light sensors, interference of the reference beam and the response beam corresponding to different path length relations.

(Prior art) *Fig. 1*

**Fig. 2**

**Fig. 3a**

*Fig. 3b*

*Fig. 4*

Receive reference beam
and response beam — 402

Receive reference beam
by first waveguide system — 404

Transport reference beam along
different path lengths to
plurality of first outputs — 406

Receiving by plurality of light
sensors a combination of
reference beam and response
beam forming interference of
reference beam and
response beam — 408

Stimultaneosly detecting by
plurality of light sensors
interference of reference beam
and response beam
corresponding to different path
length relations — 410

*Fig. 5*

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2024/129119 A1 (WASHINGTON UNIVERSITY ST LOUIS [US]) 20 June 2024 (2024-06-20)<br><br>* the whole document *<br>----- | 1-3,5,<br>7-9,<br>12-15 | INV.<br>G01B9/02015<br>A61B3/10<br>G01B9/02<br>G01B9/02091 |
| X | US 2023/160681 A1 (HAI VIDAL YEHUDA [IL] ET AL) 25 May 2023 (2023-05-25)<br>* abstract; figures 12,17 *<br>* paragraph [0208] - paragraph [0220] *<br>* paragraph [0247] - paragraph [0252] *<br>----- | 1,2,4-6,<br>9-11,15 | |
| A | US 2020/025616 A1 (AVCI BAKIYE IMRAN [NL]) 23 January 2020 (2020-01-23)<br>* paragraph [0099] - paragraph [0108]; figure 10 *<br>----- | 1-15 | |
| A | AKCA B IMRAN ET AL: "Integrated-optics based multi-beam imaging for speed improvement of OCT systems", PROGRESS IN BIOMEDICAL OPTICS AND IMAGING, SPIE - INTERNATIONAL SOCIETY FOR OPTICAL ENGINEERING, BELLINGHAM, WA, US, vol. 10056, 14 March 2017 (2017-03-14), pages 100560Q-100560Q, XP060084039, ISSN: 1605-7422, DOI: 10.1117/12.2248721 ISBN: 978-1-5106-0027-0<br>* the whole document *<br>----- | 1-15 | TECHNICAL FIELDS SEARCHED (IPC)<br><br>G01B<br>A61B |

The present search report has been drawn up for all claims

| Place of search | Date of completion of the search | Examiner |
|---|---|---|
| Munich | 16 May 2025 | Stanciu, C |

EPO FORM 1503 03.82 (P4C01)

## ANNEX TO THE EUROPEAN SEARCH REPORT
## ON EUROPEAN PATENT APPLICATION NO.

EP 24 21 6386

This annex lists the patent family members relating to the patent documents cited in the above-mentioned European search report.
The members are as contained in the European Patent Office EDP file on
The European Patent Office is in no way liable for these particulars which are merely given for the purpose of information.

16-05-2025

| Patent document cited in search report | Publication date | Patent family member(s) | | Publication date |
|---|---|---|---|---|
| WO 2024129119 A1 | 20-06-2024 | NONE | | |
| US 2023160681 A1 | 25-05-2023 | EP | 4127775 A1 | 08-02-2023 |
| | | IL | 273779 A | 31-10-2021 |
| | | US | 2023160681 A1 | 25-05-2023 |
| | | WO | 2021199027 A1 | 07-10-2021 |
| US 2020025616 A1 | 23-01-2020 | CN | 110312918 A | 08-10-2019 |
| | | EP | 3516354 A1 | 31-07-2019 |
| | | KR | 20200005524 A | 15-01-2020 |
| | | US | 2020025616 A1 | 23-01-2020 |
| | | WO | 2018055605 A1 | 29-03-2018 |

EPO FORM P0459

For more details about this annex : see Official Journal of the European Patent Office, No. 12/82